(19) Europäisches Patentamt
European Patent Office
Office européen des brevets

(11) **EP 4 353 089 A1**

(12) **EUROPEAN PATENT APPLICATION**
published in accordance with Art. 153(4) EPC

(43) Date of publication:
**17.04.2024 Bulletin 2024/16**

(21) Application number: **22816032.1**

(22) Date of filing: **30.05.2022**

(51) International Patent Classification (IPC):
**A23L 33/125** (2016.01)   **A61K 31/702** (2006.01)
**A61P 3/00** (2006.01)   **A61P 3/04** (2006.01)
**A61P 3/06** (2006.01)   **A61P 3/10** (2006.01)
**A61P 15/00** (2006.01)

(52) Cooperative Patent Classification (CPC):
**A23L 33/125; A61K 31/702; A61P 3/00;
A61P 3/04; A61P 3/06; A61P 3/10; A61P 15/00**

(86) International application number:
**PCT/JP2022/021862**

(87) International publication number:
**WO 2022/255284 (08.12.2022 Gazette 2022/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.05.2021   JP 2021091860**

(71) Applicant: **The Food Science Institute Foundation
Tokyo 192-0919 (JP)**

(72) Inventors:
• **KIMURA, Ikuo**
  **Fuchu-shi, Tokyo 183-8538 (JP)**
• **MIYAMOTO, Junki**
  **Fuchu-shi, Tokyo 183-8538 (JP)**
• **KOZUTSUMI, Daisuke**
  **Hachioji-shi, Tokyo 192-0919 (JP)**
• **TAKASUGI, Satoshi**
  **Hachioji-shi, Tokyo 192-0919 (JP)**

(74) Representative: **TBK
Bavariaring 4-6
80336 München (DE)**

(54) **ORAL ADMINISTRATION AGENT FOR PREGNANT WOMEN**

(57)    An object of the present invention is to provide an oral administration agent for a pregnant woman for used in increasing a blood short-chain fatty acid concentration in a pregnant woman or an unborn baby, imparting resistance to obesity to an unborn baby, or preventing postnatal obesity or a disorder resulting from postnatal obesity of an unborn baby, and, in order to achieve the object, the present invention provides an oral administration agent for a pregnant woman containing an oligosaccharide, for used in increasing a blood short-chain fatty acid concentration in a pregnant woman or an unborn baby, imparting resistance to obesity to an unborn baby, or preventing postnatal obesity or a disorder resulting from postnatal obesity of an unborn baby.

EP 4 353 089 A1

**Description**

TECHNICAL FIELD

The present invention relates to a novel technique for increasing a blood short-chain fatty acid concentration in a pregnant woman or an unborn baby, for imparting resistance to obesity to an unborn baby, or for preventing postnatal obesity or a disorder resulting from postnatal obesity of an unborn baby (for example, an oral administration agent for a pregnant woman).

BACKGROUND ART

In recent years, in developed countries including Japan, diseases resulting from obesity (for example, adiposity, metabolic syndrome, diabetes, hyperlipidemia, and hypertension) are increasing, and there is an increasing necessity for prevention of obesity.

Obesity occurs not only in adulthood but also in childhood (infancy, early childhood, school age, and puberty), and childhood obesity easily leads to adulthood obesity. For example, according to Annual Report of School Health Statistics Research of the Japanese Ministry of Education, Culture, Sports, Science and Technology, about 40% of school-age obesity shifts to adulthood obesity, and about 70% of puberty obesity shifts to adulthood obesity. Besides, in cases where school-age obesity shifts to adulthood obesity through puberty obesity, many of these children are obese already in early childhood. Accordingly, it is significant to prevent childhood obesity.

On the other hand, Patent Document 1 discloses a composition containing an oligosaccharide such as a fructo-oligosaccharide, and describes the use thereof for prevention and treatment of obesity.

CITATION LIST

PATENT DOCUMENT

Patent Document 1: Japanese Patent Laid-Open No. 2021-017414

SUMMARY OF INVENTION

An object of the present invention is to provide an oral administration agent for a pregnant woman, for use in increasing a blood short-chain fatty acid concentration in a pregnant woman or an unborn baby, imparting resistance to obesity to an unborn baby, or preventing postnatal obesity or a disorder resulting from postnatal obesity of an unborn baby.

Besides, another object of the present invention is to provide a method for increasing a blood short-chain fatty acid concentration in a pregnant woman or an unborn baby, imparting resistance to obesity to an unborn baby, or preventing postnatal obesity or a disorder resulting from postnatal obesity of an unborn baby, including orally administering, to a pregnant woman, an oral administration agent for a pregnant woman containing an oligosaccharide.

Still another object of the present invention is to provide use of an oral administration agent for a pregnant woman containing an oligosaccharide, for increasing a blood short-chain fatty acid concentration in a pregnant woman or an unborn baby, imparting resistance to obesity to an unborn baby, or preventing postnatal obesity or a disorder resulting from postnatal obesity of an unborn baby.

Still another object of the present invention is to provide use of an oligosaccharide in production of an oral administration agent for a pregnant woman for use in increasing a blood short-chain fatty acid concentration in a pregnant woman or an unborn baby, imparting resistance to obesity to an unborn baby, or preventing postnatal obesity or a disorder resulting from postnatal obesity of an unborn baby.

In order to solve the above problem, the present invention provides the following oral administration agent for a pregnant woman.

[A1] An oral administration agent for a pregnant woman containing an oligosaccharide, for use in increasing a blood short-chain fatty acid concentration in a pregnant woman or an unborn baby.

[A2] The oral administration agent for a pregnant woman according to [A1], wherein the oligosaccharide is a fructo-oligosaccharide.

[A3] An oral administration agent for a pregnant woman containing an oligosaccharide, for use in imparting resistance to obesity to an unborn baby.

[A4] The oral administration agent for a pregnant woman according to [A3], wherein the oligosaccharide is a fructo-oligosaccharide.

[A5] An oral administration agent for a pregnant woman containing an oligosaccharide, for use in preventing postnatal obesity, or a disorder resulting from postnatal obesity of an unborn baby.

[A6] The oral administration agent for a pregnant woman according to [A5], wherein the oligosaccharide is a fructo-oligosaccharide.

[A7] The oral administration agent for a pregnant woman according to [A5] or [A6], wherein the postnatal obesity is childhood obesity.

[A8] The oral administration agent for a pregnant woman according to [A7], wherein the childhood obesity is early-childhood obesity and/or school-age obesity.

[A9] The oral administration agent for a pregnant woman according to any one of [A5] to [A8], wherein the disorder is a disorder of glycometabolism and/or a disorder of lipid metabolism.

[A10] The oral administration agent for a pregnant woman according to [A9], wherein the disorder of glycometabolism is abnormal glucose tolerance and/or insulin resistance.

[A11] The oral administration agent for a pregnant woman according to any one of [A5] to [A8], wherein the disorder is decreased basal metabolism.

[A12] The oral administration agent for a pregnant woman according to [A11], wherein the decreased basal metabolism involves decrease in heart rate and/or decrease in body temperature.

The present invention also provides the following method.

[B1] A method for increasing a blood short-chain fatty acid concentration in a pregnant woman or an unborn baby, including orally administering, to a pregnant woman, an oral administration agent for a pregnant woman containing an oligosaccharide.

[B2] The method according to [B1], wherein the oligosaccharide is a fructo-oligosaccharide.

[B3] A method for imparting resistance to obesity to an unborn baby, including orally administering, to a pregnant woman, an oral administration agent for a pregnant woman containing an oligosaccharide.

[B4] The method according to [B3], wherein the oligosaccharide is a fructo-oligosaccharide.

[B5] A method for preventing postnatal obesity, or a disorder resulting from postnatal obesity of an unborn baby, including orally administering, to a pregnant woman, an oral administration agent for a pregnant woman containing an oligosaccharide.

[B6] The method according to [B5], wherein the oligosaccharide is a fructo-oligosaccharide.

[B7] The method according to [B5] or [B6], wherein the postnatal obesity is childhood obesity.

[B8] The method according to [B7], wherein the childhood obesity is early-childhood obesity and/or school-age obesity.

[B9] The method according to any one of [B5] to [B8], wherein the disorder is a disorder of glycometabolism and/or a disorder of lipid metabolism.

[B10] The method according to [B9], wherein the disorder of glycometabolism is abnormal glucose tolerance and/or insulin resistance.

[B11] The method according to any one of [B5] to [B8], wherein the disorder is decreased basal metabolism.

[B12] The method according to [B11], wherein the decreased basal metabolism involves decrease in heart rate and/or decrease in body temperature.

The present invention also provides the following use.

[C1] Use of an oral administration agent for a pregnant woman containing an oligosaccharide, for increasing a blood short-chain fatty acid concentration in a pregnant woman or an unborn baby.

[C2] The use according to [C1], wherein the oligosaccharide is a fructo-oligosaccharide.

[C3] Use of an oral administration agent for a pregnant woman including an oligosaccharide, for imparting resistance to obesity to an unborn baby.

[C4] The use according to [C3], wherein the oligosaccharide is a fructo-oligosaccharide.

[C5] Use of an oral administration agent for a pregnant woman including an oligosaccharide, for preventing postnatal obesity, or a disorder resulting from postnatal obesity of an unborn baby.

[C6] The use according to [C5], wherein the oligosaccharide is a fructo-oligosaccharide.

[C7] The use according to [C5] or [C6], wherein the postnatal obesity is childhood obesity.

[C8] The use according to [C7], wherein the childhood obesity is early-childhood obesity and/or school-age obesity.

[C9] The use according to any one of [C5] to [C8], wherein the disorder is a disorder of glycometabolism and/or a disorder of lipid metabolism.

[C10] The use according to [C9], wherein the disorder of glycometabolism is abnormal glucose tolerance and/or insulin resistance.

[C11] The use according to any one of [C5] to [C8], wherein the disorder is decreased basal metabolism.

[C12] The use according to [C11], wherein the decreased basal metabolism involves decrease in heart rate and/or decrease in body temperature.

The present invention also provides the following use.

[D1] Use of an oligosaccharide in production of an oral administration agent for a pregnant woman for use in increasing a blood short-chain fatty acid concentration in a pregnant woman or an unborn baby.
[D2] The use according to [D1], wherein the oligosaccharide is a fructo-oligosaccharide.
[D3] Use of an oligosaccharide in production of an oral administration agent for a pregnant woman for use in imparting resistance to obesity to an unborn baby.
[D4] The use according to [D3], wherein the oligosaccharide is a fructo-oligosaccharide.
[D5] Use of an oligosaccharide in production of an oral administration agent for a pregnant woman for use in preventing postnatal obesity, or a disorder resulting from postnatal obesity of an unborn baby.
[D6] The use according to [D5], wherein the oligosaccharide is a fructo-oligosaccharide.
[D7] The use according to [D5] or [D6], wherein the postnatal obesity is childhood obesity.
[D8] The use according to [D7], wherein the childhood obesity is early-childhood obesity and/or school-age obesity.
[D9] The use according to any one of [D5] to [D8], wherein the disorder is a disorder of glycometabolism and/or a disorder of lipid metabolism.
[D10] The use according to [D9], wherein the disorder of glycometabolism is abnormal glucose tolerance and/or insulin resistance.
[D11] The use according to any one of [D5] to [D8], wherein the disorder is decreased basal metabolism.
[D12] The use according to [D11], wherein the decreased basal metabolism involves decrease in heart rate and/or decrease in body temperature.

The present invention provides an oral administration agent for a pregnant woman for use in increasing a blood short-chain fatty acid concentration in a pregnant woman or an unborn baby, imparting resistance to obesity to an unborn baby, or preventing postnatal obesity or a disorder resulting from postnatal obesity of an unborn baby.

The present invention also provides a method for increasing a blood short-chain fatty acid concentration in a pregnant woman or an unborn baby, imparting resistance to obesity to an unborn baby, or preventing postnatal obesity or a disorder resulting from postnatal obesity of an unborn baby, including orally administering, to a pregnant woman, an oral administration agent for a pregnant woman containing an oligosaccharide.

The present invention also provides use of an oral administration agent for a pregnant woman containing an oligosaccharide, for increasing a blood short-chain fatty acid concentration in a pregnant woman or an unborn baby, imparting resistance to obesity to an unborn baby, or preventing postnatal obesity or a disorder resulting from postnatal obesity of an unborn baby.

The present invention also provides use of an oligosaccharide in production of an oral administration agent for a pregnant woman for use in increasing a blood short-chain fatty acid concentration in a pregnant woman or an unborn baby, imparting resistance to obesity to an unborn baby, or preventing postnatal obesity or a disorder resulting from postnatal obesity of an unborn baby.

DETAILED DESCRIPTION OF THE INVENTION

Hereinafter, the present invention will be described. In the present invention, the term "oral administration agent for a pregnant woman" means an oral administration agent to be orally administered to a pregnant woman, and the term "unborn baby" means an unborn baby in the womb of a pregnant woman to whom the oral administration agent for a pregnant woman of the present invention is to be orally administered.

<Active Ingredient of Oral Administration Agent for Pregnant Woman>

Hereinafter, an active ingredient of an oral administration agent for a pregnant woman of the present invention will be described.

The oral administration agent for a pregnant woman of the present invention contains an oligosaccharide. The oligosaccharide contained in the oral administration agent for a pregnant woman of the present invention may be composed of one type of oligosaccharide, or two or more types of oligosaccharides. The oral administration agent for a pregnant woman of the present invention may be composed of an oligosaccharide, or may be composed of an oligosaccharide, and one, two or more types of components other than the oligosaccharide.

An oligosaccharide means an oligomer in which 2 to 10 (for example, 3 to 7, 3 to 6, or 3 to 5) monosaccharides are bonded by a glycoside bond. Examples of the monosaccharide residue constituting the oligosaccharide include a glucose residue, a fructose residue, and a galactose residue. The oligosaccharide may be composed of one type of monosaccharide residue, or two or more types of monosaccharide residues.

Examples of the oligosaccharide include a fructo-oligosaccharide, a galacto-oligosaccharide, an isomalto-oligosaccharide, a xylo-oligosaccharide, a gentio-oligosaccharide, a lactosucrose, a fucosyllactose, and a nigero-oligosaccharide, among which a fructo-oligosaccharide is preferred.

The fructo-oligosaccharide means an oligomer in which 1 to 3 fructoses are β-2,1-glycosidically bonded to a fructose residue of sucrose.

Examples of the fructo-oligosaccharide include 1-kestose (GF2), nystose (GF3), and 1F-β-fructofuranosylnystose (GF4).

The fructo-oligosaccharide may be one selected from GF2, GF3, and GF4, or may be a mixture of two or more selected from GF2, GF3, and GF4.

The fructo-oligosaccharide may be produced from a raw material of lactose and sucrose with β-fructofuranosidase, or may be produced by hydrolysis of inulin. The fructo-oligosaccharide may be a commercially available product such as Mei-oligo(TM) P manufactured by Meiji Food Materia Co., Ltd.

An oligosaccharide content in the oral administration agent for a pregnant woman of the present invention can be appropriately adjusted in consideration of the form of the oral administration agent for a pregnant woman of the present invention, the number of doses per day of the oral administration agent for a pregnant woman of the present invention, a daily dose of the oral administration agent for a pregnant woman of the present invention, and the like. The oligosaccharide content in the oral administration agent for a pregnant woman of the present invention can be adjusted in a range of, for example, 1 to 99% by mass, based on the mass of the oral administration agent for a pregnant woman of the present invention, and is preferably adjusted to 10 to 95% by mass, and more preferably adjusted to 20 to 50% by mass. When the oral administration agent for a pregnant woman of the present invention contains two or more types of oligosaccharides, the oligosaccharide content in the oral administration agent for a pregnant woman of the present invention means a total content of the two or more types of oligosaccharides.

<Use of Oral Administration Agent for Pregnant Woman>

Hereinafter, use of the oral administration agent for a pregnant woman of the present invention will be described.

In a first embodiment, the oral administration agent for a pregnant woman of the present invention is used for increasing a blood short-chain fatty acid concentration in a pregnant woman or an unborn baby. The oral administration agent for a pregnant woman of the present invention may be used for increasing a blood concentration of one type of short-chain fatty acid, or for increasing blood concentrations of two or more types of short-chain fatty acids. Here, the term "increasing a blood short-chain fatty acid concentration in a pregnant woman or an unborn baby" means that a blood short-chain fatty acid concentration in a pregnant woman or an unborn baby after oral administration of the oral administration agent for a pregnant woman of the present invention is increased as compared with a blood short-chain fatty acid concentration in the pregnant woman or the unborn baby before oral administration of the oral administration agent for a pregnant woman of the present invention. When a blood short-chain fatty acid concentration in a pregnant woman or an unborn baby after oral administration of the oral administration agent for a pregnant woman of the present invention is significantly increased as compared with a blood short-chain fatty acid concentration in the pregnant woman or the unborn baby before oral administration of the oral administration agent for a pregnant woman of the present invention, for example, when a change ratio of a blood short-chain fatty acid concentration in a pregnant woman or an unborn baby before and after oral administration of the oral administration agent for a pregnant woman of the present invention is preferably 5% or more, more preferably 10% or more, still more preferably 15% or more, and particularly more preferably 20% or more, it can be determined that the blood short-chain fatty acid concentration in the pregnant woman or the unborn baby is increased. As for a pregnant woman, the blood short-chain fatty acid concentrations in the pregnant woman obtained before and after oral administration of the oral administration agent for a pregnant woman of the present invention are compared, and as for an unborn baby, the blood short-chain fatty acid concentrations in the unborn baby obtained before and after oral administration of the oral administration agent for a pregnant woman of the present invention are compared. The change ratio of the blood short-chain fatty acid concentration is calculated based on the following formula:

$$\text{Change ratio of blood short-chain fatty acid concentration} = \frac{(\text{blood short-chain fatty acid concentration after oral administration} - \text{blood short-chain fatty acid concentration before oral administration})}{\text{blood short-chain fatty acid concentration before administration}} \times 100$$

When the oral administration agent for a pregnant woman of the present invention is administered to a pregnant woman, a blood short-chain fatty acid concentration in the pregnant woman and her unborn baby is increased. A short-chain fatty acid is a principal metabolite of intestinal bacteria, but an unborn baby does not have intestinal bacteria, and hence it is presumed that, due to the increase of the blood short-chain fatty acid concentration in a pregnant woman, the blood short-chain fatty acid concentration in her unborn baby is increased. Accordingly, when the blood short-chain fatty acid concentration in a pregnant woman is increased, it can be determined that the blood short-chain fatty acid concentration in her unborn baby is also increased.

The short-chain fatty acid is preferably a chain monocarboxylic acid. The chain may be a linear chain or a branched chain.

The number of carbon atoms of the short-chain fatty acid is preferably 2 to 6, more preferably 2 to 4, and still more preferably 2 to 3.

Examples of the short-chain fatty acid include acetic acid, propionic acid, butyric acid, isobutyric acid, valeric acid, isovaleric acid, and caproic acid, among which acetic acid, propionic acid, and butyric acid are preferred, and acetic acid and propionic acid are more preferred.

In a second embodiment, the oral administration agent for a pregnant woman of the present invention is used for imparting resistance to obesity to an unborn baby, or for preventing postnatal obesity or a disorder resulting from postnatal obesity of an unborn baby. Here, the term "preventing postnatal obesity or a disorder resulting from postnatal obesity of an unborn baby" encompasses preventing, suppressing, or delaying onset or exacerbation of postnatal obesity or a disorder resulting from postnatal obesity in the unborn baby, and reducing risk of onset or exacerbation of postnatal obesity or a disorder resulting from postnatal obesity in the unborn baby. Besides, the term "preventing a disorder resulting from postnatal obesity of an unborn baby" encompasses preventing a disorder resulting from postnatal obesity of an unborn baby by preventing postnatal obesity of the unborn baby, and preventing a disorder resulting from postnatal obesity of an unborn baby after onset of postnatal obesity of the unborn baby.

When the oral administration agent for a pregnant woman of the present invention is administered to a pregnant woman, the blood short-chain fatty acid concentration in the pregnant woman and her unborn baby is increased. The short-chain fatty acid is not only used as an energy source in a living body but also involved in physiological function as a signaling molecule. For example, when a short-chain fatty acid acts on a short-chain fatty acid receptor such as GPR41, or GPR43, actions of production increase of PYY (peptide YY), that is, appetite suppressing hormone, production increase of GLP-1 (glucagon-like peptide-1) that increases insulin sensitivity, increase of energy consumption, suppression of adipocyte hypertrophy, and the like are exhibited (Junki Miyamoto, et al., The Lipid, Vol. 27, No. 2, 2016-4). Accordingly, it is presumed that, when the short-chain fatty acid receptor such as GPR41, or GPR43 is activated by the increase of the blood short-chain fatty acid concentration in an unborn baby, metabolism (glycometabolism, lipid metabolism or the like), the physiological functions of the endocrine system and the like are improved, and thus resistance to obesity (tendency to be less likely to be obese) is imparted to the unborn baby, and as a result, postnatal obesity or a disorder resulting from postnatal obesity of the unborn baby is prevented. In recent years, it has been reported that nutrient environment in a period when sensitivity of organ formation and maturation (namely, plasticity) is high (fetal period and early postnatal period) largely affects postnatal metabolic system (namely, metabolic programming). The resistance to obesity imparted to an unborn baby by the increase of the blood short-chain fatty acid concentration (tendency to be less likely to be obese) is presumed to be one aspect of such metabolic programming. It is presumed that the increase in the blood short-chain fatty acid concentration in an unborn baby results in an increase in muscle mass, a decrease in the number of white adipocytes, an increase in the number of brown adipocytes and the like in the unborn baby, and thus resistance to obesity (tendency to be less likely to be obese) is imparted to the unborn baby. It is presumed that, when resistance to obesity (tendency to be less likely to be obese) is imparted to an unborn baby, the basal metabolism (including maintenance of a normal heart rate, and maintenance of a normal body temperature) after the birth of the unborn baby is improved, and such improvement of the basal metabolism after birth of the unborn baby also contributes to the prevention of postnatal obesity or a disorder resulting from postnatal obesity of the unborn baby.

Obesity means a state where adipose tissues are excessively accumulated.

The obesity is classified, in accordance with a site where adipose tissues are accumulated, into subcutaneous fat obesity and visceral fat obesity. In the second embodiment, the obesity may be either of subcutaneous fat obesity and visceral fat obesity.

The obesity is classified into primary obesity (also designated as simple obesity) and secondary obesity. In the second embodiment, the obesity is usually primary obesity. Primary obesity is caused, for example, due to excessive energy intake (for example, continuous intake of a high calorie diet, a high fat diet, and the like).

A high calorie diet is, for example, a diet having a calorie exceeding estimated energy requirement (kcal/day) of a target who ingests the high calorie diet. A high fat diet is, for example, a diet exceeding a dietary reference intake of lipid (% energy) of a target who ingests the high fat diet. As for the estimated energy requirement and the dietary reference intake of lipid of a target, "Dietary Reference Intakes for Japanese (2020)" determined by the Japanese Ministry of Health, Labor and Welfare can be referred to.

Postnatal obesity means obesity of an unborn baby in future (after birth).

Examples of the disorder resulting from postnatal obesity include a disorder of glycometabolism, a disorder of lipid metabolism, and decreased basal metabolism. The disorder to be prevented may be one or two or more of these.

Examples of the disorder of glycometabolism include abnormal glucose tolerance, and insulin resistance. The disorder to be prevented may be one or two or more of these. Accordingly, prevention of the disorder resulting from postnatal obesity of an unborn baby encompasses reduction of onset risk of abnormal glucose tolerance, suppression of excessive increase of blood glucose level, improvement or prevention of exacerbation of insulin resistance, and the like, in the unborn baby after the birth.

Examples of the disorder of lipid metabolism include increase of a blood total cholesterol (TC) concentration, increase of a blood low-density lipoprotein cholesterol (LDL-C) concentration, increase of a blood triglyceride (neutral fat, Tg) concentration, and decrease of a blood high-density lipoprotein cholesterol (HDL-C) concentration. The disorder to be prevented may be one or two or more of these.

The decreased basal metabolism involves, for example, one of or two or more of decrease in heart rate, decrease in body temperature, and the like. Accordingly, the prevention of the disorder resulting from postnatal obesity of an unborn baby encompasses maintenance of normal heart rate, maintenance of normal body temperature, and the like.

The disorder resulting from postnatal obesity encompasses diseases resulting from the postnatal obesity. Examples of the diseases resulting from the postnatal obesity include hypertension, type 2 diabetes, early arteriosclerosis, nonalcoholic fatty liver disease (NAFLD), hyperinsulinemia, acanthosis nigricans, hyper-TC-cholesterolemia, hyper non-HDL-C cholesterolemia, hypertriglyceridemia, low HDL-cholesterolemia, and hyperuricemia. The disease to be prevented may be one or two or more of these.

The diseases resulting from postnatal obesity include adiposity and metabolic syndrome. The disease to be prevented may be one or two of these. Adiposity is a state where health disorders (medical abnormalities) resulting from or related to obesity are complicated or these health disorders are expected, and treatment (medical intervention) for medically reducing obesity is required. Metabolic syndrome is a state essentially involving abdominal obesity where two or more of three artery risk factors of dyslipidemia (hypertriglyceridemia, low HDL-C cholesterolemia), blood pressure elevation, and fasting hyperglycemia are accumulated in a specific individual.

The postnatal obesity is not especially limited as long as it is obesity occurring in future (after birth) of an unbody baby, and may be obesity occurring in any period after the birth, and is preferably childhood obesity. Childhood obesity easily leads to adulthood obesity. Accordingly, it is significant to prevent childhood obesity.

Childhood obesity is classified, in accordance with timing of onset of obesity, into infancy obesity, early-childhood obesity, school-age obesity, and puberty obesity. Childhood means a period from the birth to an age younger than 18 years old, infancy means a period from the birth to an age younger than 1 year old, early childhood means a period from an age of 1 year old to an age younger than 6 years old, school age means a period from an age of 6 years old to an age younger than 12 years old, and puberty means a period from an age of 12 years old to an age younger than 18 years old.

In examples described below, mice are used. A mouse is presumed to age about 30 times faster than a human. Accordingly, it is presumed that the infancy of a human corresponds to a period from the birth up to about 2 weeks old in a mouse, that the early childhood of a human corresponds to a period from about 2 weeks old up to about 10 weeks old in a mouse, that the school age of a human corresponds to a period from about 10 weeks old up to about 21 weeks old in a mouse, and that the puberty of a human corresponds to a period from about 21 weeks old up to about 31 weeks old in a mouse.

Most cases of infancy obesity are physiological obesity naturally decreased in early childhood, and are presumed to less relate to obesity in future. On the other hand, early-childhood obesity easily leads to school-age obesity, school-age obesity easily leads to puberty obesity, and puberty obesity easily leads to adulthood obesity. Accordingly, the postnatal obesity to be prevented in the second embodiment is preferably one or more selected from early-childhood obesity, school-age obesity, and puberty obesity, and more preferably one or more selected from early-childhood obesity, and school-age obesity, and more preferably early-childhood obesity. It is difficult to make children in early childhood continue exercise with prescribed or higher intensity for a prescribed or longer period, and therefore, obesity is easily caused due to influence of food (for example, excessive baby food, excessive snacks and the like), and lifestyle (for example, lack of exercise because of increased time of indoor play due to development of TV, games, Internet and the like.). According to a recent report, it has been reported that a person suffering from obesity in his/her puberty has worse life, medical care, and life prognosis after old age and beyond compared with a person not suffering from obesity in his/her puberty. Besides, most cases of puberty obesity are continued from school-age obesity. Accordingly, it is very significant to prevent one or more selected from early-childhood obesity, school-age obesity, and puberty obesity, and particularly one or more selected from early-childhood obesity, and school-age obesity.

The early childhood is divided into a first half (1 year old or older and younger than 3 years old), and a second half (3 years old or older and younger than 6 years old). The obesity in the early childhood to be prevented in the second embodiment is preferably obesity in the first half of the early childhood. Weaning is usually completed from about 1 year old to 1 year and 6 months old, and in the first half of the early childhood after completing weaning, there are increased opportunities of intake of a high calorie diet, a high fat diet, and the like. Besides, when swallowing and fast eating are habituated in the first half of the early childhood after completing weaning, risk of obesity increases. Accordingly, it is significant to prevent obesity in the first half of early childhood.

Childhood obesity can be determined, for example, based on "Guidelines for the management of obesity disease in children and adolescents 2017" (edited by Japan Society For The Study Of Obesity, issued by Life Science Publishing Co., Ltd., April 14, 2017). It is noted that targets of "Guidelines for the management of obesity disease in children and adolescents 2017" are children of 6 years old or older and younger than 18 years old.

Early-childhood Obesity can be determined, for example, based on "Guideline for Early-childhood Obesity" (Japan Pediatric Society, nutrition committee, childhood obesity subcommittee, March 2019).

For determination of obesity, for example, a degree of obesity is used as an index, and in early childhood, a degree of obesity of 15% or more is determined as obesity, and in school age or later, for example, a degree of obesity of 20% or more is determined as obesity. It is noted that the degree of obesity is calculated based on the following formula:

$$\text{Degree of obesity (\%) = (actual weight - standard weight) / standard weight} \times 100$$

<Usage and Dosage of Oral Administration Agent for Pregnant Woman>

Hereinafter, the usage and the dosage of the oral administration agent for a pregnant woman of the present invention will be described.

A pregnant woman to ingest the oral administration agent for a pregnant woman of the present invention may be a pregnant woman at any stage from gestation to delivery, and is preferably a pregnant woman in the 4th week or later of gestation, more preferably a pregnant woman in the 16th week or later of gestation, still more preferably a pregnant woman in the 28th week or later of gestation, and particularly more preferably a pregnant woman in the 36th week or later of gestation. Obesity is a state where adipose tissues are excessively accumulated, and an adipose tissue is composed of adipocytes. The adipocytes are classified into two types, white adipocytes and brown adipocytes. The white adipocytes store excessive energy in the form of triglyceride (produced in the liver). Excessive increase of white adipocytes can be obesity. It is known that the number of adipocytes increases in 3 periods of fetal period (end of pregnancy), infancy, and puberty. Accordingly, it is preferable that a pregnant woman ingests the oral administration agent for a pregnant woman of the present invention in the whole gestation period. In particular, when the oral administration agent for a pregnant woman of the present invention is ingested at the end of gestation (the 28th week or later of gestation), excessive increase of adipocytes in the fetal period can be suppressed.

An administration period of the oral administration agent for a pregnant woman of the present invention is preferably 10 weeks or more, more preferably 12 weeks or more, and still more preferably 40 weeks or more. The upper limit of the administration period is not especially limited. It is preferable for a pregnant woman to ingest the oral administration agent for a pregnant woman of the present invention in the whole gestation period. It is preferable for a pregnant woman to ingest the oral administration agent for a pregnant woman of the present invention at least for about 3 months (12 weeks) or more at the end of pregnancy corresponding to a period when adipocytes increase in the unborn baby.

The number of days of administration per week of the oral administration agent for a pregnant woman of the present invention is preferably 1 day or more per week, more preferably 3 days or more per week, and still more preferably 7 days per week (namely, every day).

A frequency of administration per day of the oral administration agent for a pregnant woman of the present invention is not especially limited, and for example, once to several times (for example, twice, three times or the like) per day.

A dose of the oligosaccharide per day by the administration of the oral administration agent for a pregnant woman of the present invention is preferably 1 to 20 g, more preferably 1.5 to 15 g, still more preferably 2 to 10 g, and particularly more preferably 3 to 7 g. When the oral administration agent for a pregnant woman of the present invention contains two or more types of oligosaccharides, the dose of the oligosaccharide means a total dose of the two or more types of oligosaccharides. It is noted that a dose of the oligosaccharide required for obtaining a desired effect (required dose) can be obtained by converting a required dose obtained in an animal experiment (such as a mouse experiment) to a required dose for a human by using the following formula based on documents provided by Food Safety Commission of Japan, or may be obtained by converting human equivalent dose (HED) 12.3 based on a body surface area of a mouse into a required dose for a human:

$$\text{Required dose for human (equivalent) (g/kg of body weight)} =$$
$$\text{(required dose for animal (g/kg of body weight)) / (safety factor)}$$

It is noted that the safety factor in the formula is 100.

<Form of Oral Administration Agent for Pregnant Woman>

Hereinafter, a form of the oral administration agent for a pregnant woman of the present invention will be described.

The oral administration agent for a pregnant woman of the present invention is preferably a composition, and more preferably a food composition or a pharmaceutical composition.

Examples of the food composition include health food, nutritional supplement, functional food, food with health claims (such as food for specified health uses, food with nutrient function claims, and food with functional claims), and special purpose food (such as a milk powder for a pregnant and nursing woman, or a lactating woman).

Examples of a form of the food composition includes forms of a solid, a powder, a paste, a semi-liquid, a gel, and a liquid.

The food composition is not especially limited as long as it is food or drink capable of containing an oligosaccharide, and examples include instant food such as instant noodles, retort food, canned food, microwave food, instant soups/miso soups, and freeze-dried food; drinks such as a cooling drink, a fruit juice drink, a vegetable juice drink, a soy milk drink, a coffee drink, a tea drink, a powdered drink, a concentrated drink, and an alcoholic drink; flour products such as bread, pasta, noodles, a cake mix, and bread crumbs; sweets such as a hard candy, a caramel, a chewing gum, chocolate, a cookie, a biscuit, a bar, a cake, a pie, snack, a cracker, a Japanese sweet, a mousse, and a dessert confectionery; seasonings such as a sauce, a tomato processing seasoning, a flavor seasoning, a cooking mix, a tare sauce, a dressing, a soup base, and a curry/stew base; fats and oils such as a processed oil, a butter, a margarine, and a mayonnaise; dairy products such as a milk drink, a milky drink, fermented milk, a cheese, a yogurt, a lactic acid bacteria beverage, a milky drink, a cheese, an ice cream, a cream, a baby formula, a liquid milk, and a powdered milk; agricultural products such as canned agricultural food, a jam/marmalade, and a cereal food; and frozen food, a liquid diet, and a supplement. The food composition can be produced by a usual production method except that the oligosaccharide is contained.

A component contained in the food composition in addition to the oligosaccharide is not especially limited. Examples of the component used in addition to the oligosaccharide include water, proteins, sugars, lipids, vitamins, minerals, organic acids, organic bases, fruit juices, and flavors. Examples of the proteins include animal and vegetable proteins such as a whole milk powder, a skimmed milk powder, a partially skimmed milk powder, casein, a whey powder, whey protein, a whey protein concentrate, a whey protein isolate, $\alpha$-casein, $\beta$-casein, $\kappa$-casein, $\beta$-lactoglobulin, $\alpha$-lactalbumin, lactoferrin, soy protein, egg protein, meat protein, and hydrolysates thereof, and various milk derived components such as butter, a whey mineral, a cream, whey, non-protein nitrogen, sialic acid, phospholipid, and lactose. Examples of the sugars include general sugars, modified starches (such as dextrin, soluble starch, British starch, oxidized starch, starch ester, and starch ether), and dietary fiber. Examples of the lipids include animal fats such as lard, fish oil, and fractionated oils, hydrogenated oils, and transesterified oil thereof; and vegetable fats such as palm oil, safflower oil, corn oil, rapeseed oil, coconut oil, and fractionated oils, hydrogenated oils, and transesterified oil thereof. Examples of the vitamins include vitamin A, carotens, vitamin B group, vitamin C, vitamin D group, vitamin E, vitamin K group, vitamin P, vitamin Q, niacin, nicotinic acid, pantothenic acid, biotin, inositol, choline, and folic acid, and examples of the minerals include calcium, potassium, magnesium, sodium, copper, iron, manganese, zinc, selenium, and whey mineral. Examples of the organic acids include malic acid, citric acid, butyric acid, and tartaric acid. One of these components may be singly used, or two or more of these may be used in combination.

Examples of the pharmaceutical composition include a granule, a powder, a tablet (including a sugar-coated tablet), a pill, a capsule, a syrup, an emulsion, and a suspension. These formulations can be produced by an ordinary method using a pharmaceutically acceptable carrier. Examples of the pharmaceutically acceptable carrier include an excipient, a binder, a diluent, an additive, a perfume, a buffer, a thickener, a colorant, a stabilizer, an emulsifier, a dispersant, a suspending agent, and a preservative. One of these pharmaceutically acceptable carriers may be singly used, or two or more of these may be used in combination.

Examples of the carrier include water, a pharmaceutically acceptable organic solvent, collagen, polyvinyl alcohol, polyvinyl pyrrolidone, a carboxyvinyl polymer, sodium alginate, water-soluble dextran, water-soluble dextrin, carboxymethyl starch sodium, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, lactose, a pharmaceutically acceptable surfactant, and an artificial cell structure such as liposome.

The oral administration agent for a pregnant woman of the present invention may be packaged so that every dose can be easily administered. One dose may be packaged in a single pack, or may be packed in a plurality of packs. When provided in a packaged form, the oral administration agent is preferably provided in the form of a set consisting of doses to be ingested in a prescribed period (for example, doses to be ingested for several days). The packaged form is not especially limited as long as a prescribed amount can be prescribed, and examples include wrapping paper, a bag, a soft bag, a paper container, a can, a bottle, and a capsule.

The use, the dosage, the usage and the like of the oral administration agent for a pregnant woman of the present invention may be displayed on a package, a container, or a package insert (such as product description, a brochure, a promotion item, or a brand site). Here, the term "display" encompasses all forms of display for informing consumers of the above-described use. This display may be any form of display for recalling/analogizing the above-described use, dosage and usage, and can encompass all displays regardless of the purpose of the display, the content of the display, a matter used for the display, a medium and the like.

EXAMPLES

[Test Example 1]

C57BL/6J female mice were divided into a group A (n = 3) and a group B (n = 3). From the 1st day of gestation to the delivery date, the female mice of group A were fed with a control diet, and the female mice of group B were fed with a fructo-oligosaccharide diet. As the control diet, one obtained by mixing a feed according to AIN-93G (standard purified diet proposed by the American Institute of Nutrition (AIN) in 1933 for use in nutritional studies using mice and rats) with 10% by mass of cellulose was used, and as the fructo-oligosaccharide diet, one obtained by mixing a feed according to AIN-93G with 10% by mass of a fructo-oligosaccharide. As the fructo-oligosaccharide, Mei-oligo(TM) P manufactured by Meiji Food Materia Co., Ltd. was used.

Neonatal mice born from the female mice of group A and group B were raised until weaning by temporary mothers that ingested a normal diet (CE-2, manufactured by CLEA Japan, Inc.). After the neonatal mice born from the female mice of group A and group B were weaned at 4 weeks of age, a high fat diet was ingested to these mice for 12 weeks to induce obesity (n = 8 for neonatal mice born from the female mice of group A, and n = 7 for neonatal mice born from the female mice of group B). As the high fat diet, D12492 manufactured by Research Diets was used.

<Weight>

For 12 weeks after weaning, the weight of each neonatal mouse at each week of age was measured. Measurement results of the weight (g) are shown in Table 1.

[Table 1]

| Table 1: Body Weight | | | | | |
|---|---|---|---|---|---|
| | Body weight (g) | | | | |
| | Neonatal mice born from female mice of group A (n = 8) | | Neonatal mice born from female mice of group B (n = 7) | | Student's t test |
| | Average | Standard error | Average | Standard error | p value |
| 4 weeks of age | 30.74 | 1.305 | 27.33 | 1.649 | 0.1249 |
| 5 weeks of age | 37.07 | 1.640 | 34.94 | 1.352 | 0.3445 |
| 6 weeks of age | 43.73 | 2.188 | 40.79 | 1.382 | 0.2923 |
| 7 weeks of age | 47.59 | 2.600 | 44.74 | 1.438 | 0.3742 |
| 8 weeks of age | 50.53 | 2.754 | 47.36 | 1.813 | 0.3679 |
| 9 weeks of age | 53.11 | 2.545 | 49.33 | 1.940 | 0.2697 |

(continued)

| Table 1: Body Weight | | | | | |
|---|---|---|---|---|---|
| | Body weight (g) | | | | |
| | Neonatal mice born from female mice of group A (n = 8) | | Neonatal mice born from female mice of group B (n = 7) | | Student's t test |
| | Average | Standard error | Average | Standard error | p value |
| 10 weeks of age | 56.04 | 2.681 | 51.88 | 1.835 | 0.2364 |
| 11 weeks of age | 59.85 | 3.061 | 55.75 | 2.000 | 0.2982 |
| 12 weeks of age | 63.91 | 3.065 | 58.53 | 2.273 | 0.1925 |
| 13 weeks of age | 67.00 | 2.738 | 60.81, | 2.485 | 0.1221 |
| 14 weeks of age | 69.41 | 2.692 | 61.60 | 2.728 | 0.06331 |
| 15 weeks of age | 70.22 | 2.193 | 62.34 | 2.717 | 0.04014 |
| 16 weeks of age | 71.30 | 2.012 | 61.83 | 2.545 | 0.01116 |

As shown in Table 1, the weight increase due to the high fat diet was significantly suppressed in the neonatal mice born from the female mice of group B as compared with the neonatal mice born from the female mice of group A.

<Heart Rate and Body Temperature>

The high fat diet was ingested to the mice for 12 weeks after weaning, and then the heart rate and the body temperature of each neonatal mouse were measured (n = 8 for neonatal mice born from the female mice of group A, and n = 7 for neonatal mice born from the female mice of group B). Measurement results of the heart rate (beats/min) and the body temperature (°C) are shown in Table 2.

The heart rate was measured by tail cuff method using a non-preheating, non-invasive blood pressure monitor for mice and rats "MK-2000ST" manufactured by Muromachi Kikai Co., Ltd.

The body temperature was measured with a pocket reader ("GLOBAL POCKET READER (Bio-Thermo)" manufactured by Destron Fearing Corporation) with a chip for temperature measurement ("Life Chip(TM) with Bio-Thermo Technology" manufactured by Destron Fearing Corporation) implanted on the back of each neonatal mouse.

[Table 2]

| Table 2: Heart Rate and Body Temperature | | | | | |
|---|---|---|---|---|---|
| | Neonatal mice born from female mice of group A (n = 8) | | Neonatal mice born from female mice of group B (n = 7) | | Student's t test |
| | Average | Standard error | Average | Standard error | p value |
| Heart rate (beats/min) | 665.0 | 8.292 | 701.6 | 9.569 | 0.01231 |
| Body temperature (°C) | 36.3 | 0.08332 | 37.1 | 0.1345 | 0.000197 |

As shown in Table 2, decrease in the heart rate and decrease in the body temperature due to the high fat diet were significantly suppressed in the neonatal mice born from the female mice of group B as compared with the neonatal mice born from the female mice of group A.

<Metabolic Parameters>

After measuring the heart rate and the body temperature, metabolic parameters (fasting blood glucose level, glucose tolerance, and insulin sensitivity) of the neonatal mice were measured (n = 8 for neonatal mice born from the female mice of group A, and n = 7 for neonatal mice born from the female mice of group B). Measurement results of the metabolic parameters are shown in Tables 3 to 5.

The fasting blood glucose level was measured as follows: Blood was collected from a tail vein of each neonatal mouse having been fasted for 5 hours to measure blood glucose level (mg/dL) with a glucose kit for self-test "LFS Quick Sensor"(TR) manufactured by Life Scan Japan.

The glucose tolerance was measured as follows: To each neonatal mouse having been fasted for 16 hours, 2 mg/g of body weight of glucose (manufactured by FUJIFILM Wako Pure Chemical Corporation) was intraperitoneally administered, and blood was collected from a tail vein of the neonatal mouse 0 minute, 15 minutes, 30 minutes, 60 minutes, and 120 minutes after the glucose administration to measure blood glucose level (mg/dL) with a glucose kit for self-test "LFS Quick Sensor"(TR) manufactured by Life Scan Japan.

The insulin sensitivity was measured as follows: To each neonatal mouse having been fasted for 3 hours, 3 mU/g of body weight of insulin (manufactured by Sigma) was intraperitoneally administered, and blood was collected from a tail vein of the neonatal mouse 0 minute, 15 minutes, 30 minutes, 60 minutes, and 120 minutes after the insulin administration to measure blood glucose level (mg/dL) with a glucose kit for self-test "LFS Quick Sensor"(TR) manufactured by Life Scan Japan.

[Table 3]

| Table 3: Fasting Blood Glucose Level | | | | |
|---|---|---|---|---|
| Glucose level (mg/dL) | | | | |
| Neonatal mice born from female mice of group A (n = 8) | | Neonatal mice born from female mice of group B (n = 7) | | Student's t test |
| Average | Standard error | Average | Standard error | p value |
| 243.9 | 8.727 | 181.0 | 2.545 | 0.0001107 |

[Table 4]

| Table 4: Glucose Tolerance | | | | | |
|---|---|---|---|---|---|
| After glucose administration | Glucose level (mq/dL) | | | | |
| | Neonatal mice born from female mice of group A (n = 8) | | Neonatal mice born from female mice of group B (n = 7) | | Student's t test |
| | Average | Standard error | Average | Standard error | p value |
| 0 min | 85.13 | 3.568 | 81.50 | 4.698 | 0.5488 |
| 15 min | 367.0 | 17.69 | 295.3 | 12.72 | 0.005339 |
| 30 min | 412.1 | 24.83 | 344.9 | 14.34 | 0.03428 |
| 60 min | 357.8 | 16.44 | 324.9 | 22.64 | 0.2596 |
| 120 min | 223.4 | 13.14 | 220.9 | 16.92 | 0.9088 |

[Table 5]

Table 5: Insulin Sensitivity

| After insulin administration | Glucose level (mg/dL) | | | | |
|---|---|---|---|---|---|
| | Neonatal mice born from female mice of group A (n = 8) | | Neonatal mice born from female mice of group B (n = 7) | | Student's t test |
| | Average | Standard error | Average | Standard error | p value |
| 0 min | 167.1 | 10.43 | 163.6 | 9.183 | 0.8048 |
| 15 min | 136.5 | 16.80 | 118.6 | 11.57 | 0.3956 |
| 30 min | 118.9 | 12.75 | 84.63 | 6.158 | 0.02974 |
| 60 min | 79.25 | 6.397 | 51.63 | 11.00 | 0.04768 |
| 120 min | 86.50 | 7.778 | 94.63 | 11.48 | 0.5673 |

As shown in Table 3, elevation of fasting blood glucose level was significantly suppressed in the neonatal mice born from the female mice of group B as compared with the neonatal mice born from the female mice of group A.

As shown in Table 4, abnormal glucose tolerance was significantly suppressed in the neonatal mice born from the female mice of group B as compared with the neonatal mice born from the female mice of group A.

As shown in Table 5, decrease of insulin sensitivity (namely, insulin resistance) was significantly suppressed in the neonatal mice born from the female mice of group B as compared with the neonatal mice born from the female mice of group A.

<Tissue Weight>

After measuring the metabolic parameters, each neonatal mouse was dissected to measure tissue weight (peri-testicular adipose tissue, perirenal adipose tissue, subcutaneous adipose tissue, and liver) (n = 8 for neonatal mice born from the female mice of group A, and n = 7 for neonatal mice born from the female mice of group B). Measurement results of the tissue weight (g) are shown in Table 6.

[Table 6]

Table 6: Tissue Weight

| | Tissue weight (g) | | | | |
|---|---|---|---|---|---|
| | Neonatal mice born from female mice of group A (n = 8) | | Neonatal mice born from female mice of group B (n = 7) | | Student's t test |
| | Average | Standard error | Average | Standard error | p value |
| epi | 1.428 | 0.1926 | 1.182 | 0.09051 | 0.2913 |
| peri | 1.087 | 0.09471 | 0.7933 | 0.08155 | 0.03746 |
| sub | 1.439 | 0.1277 | 0.9581 | 0.1146 | 0.01597 |
| liver | 3.020 | 0.2784 | 2.740 | 0.2751 | 0.4908 |
| epi: peri-testicular adipose tissue, peri: perirenal adipose tissue, sub: subcutaneous adipose tissue, liver: liver | | | | | |

As shown in Table 6, accumulation of the subcutaneous and visceral adipose tissues was significantly suppressed in the neonatal mice born from the female mice of group B as compared with the neonatal mice born from the female mice of group A.

[Test Example 2]

C57BL/6J female mice were divided into group A (n = 4) and group B (n = 3). From the 1st day of gestation, the female mice of group A were fed with the same control diet as that used in Test Example 1, and the female mice of group B were fed with the same fructo-oligosaccharide diet as that used in Test Example 1.

On the 18.5th day of gestation, the female mice were dissected, and a blood acetic acid concentration and a blood propionic acid concentration were measured in the female mice of group A, unborn babies of the female mice of group A, the female mice of group B, and unborn babies of the female mice of group B (n = 4 for the female mice of group A, n = 7 for unborn mice obtained from the female mice of group A, n = 3 for the female mice of group B, and n = 6 for unborn mice obtained from the female mice of group B). Measurement results of the blood acetic acid concentration (μM) and the blood propionic acid concentration (μM) are shown in Tables 7 and 8.

The blood acetic acid concentration and the blood propionic acid concentration were measured as follows: 5-Sulfosalicylic acid (manufactured by FUJIFILM Wako Pure Chemical Corporation) was added to plasma, the resultant was allowed to stand still on ice for 15 minutes, and then centrifuged at 4°C and 15,000 x g for 15 minutes to collect a supernatant. Hydrochloric acid and diethyl ether were added to the supernatant, followed by vortex mixing for 5 minutes. The resultant was centrifuged at room temperature and 3,000 x g for 5 minutes, followed by collecting an ether layer. Diethyl ether was added again to the collected ether layer, and the resultant was centrifuged at room temperature and 3,000 x g for 5 minutes, followed by collecting an ether layer. The thus collected ether layer was used as a sample. An acetic acid concentration and a propionic acid concentration in the sample were measured, using 2-ethylbutyric acid as an internal standard, with a measurement apparatus of GC/MS ("GCMS-QP2010 Ultra" manufactured by Shimadzu Corporation) using "VF-WAXms column" manufactured by Agilent Technologies, Inc. as a column. For the measurement method, Kimura et al., Science 367 (6481), eaaw8429 (2020) and Miyamoto et al., PNAS 116 (47) 23813-23821 (2019) were referred to.

[Table 7]

| Table 7: Blood Acetic Acid Concentration and Blood Propionic Acid Concentration | | | | | |
|---|---|---|---|---|---|
| | Female mice of group A (n = 4) | | Female mice of group B (n = 3) | | Student's t test |
| | Average | Standard error | Average | Standard error | p value |
| Blood acetic acid concentration (μM) | 379.2 | 5.996 | 505.2 | 8.988 | 0.00006587 |
| Blood propionic acid concentration (μM) | 88.33 | 2.803 | 101.6 | 4.439 | 0.04433 |

[Table 8]

| Table 8: Blood Acetic Acid Concentration and Blood Propionic Acid Concentration | | | | | |
|---|---|---|---|---|---|
| | Unborn babies of female mice of group A (n = 7) | | Unborn babies of female mice of group B (n = 6) | | Student's t test |
| | Average | Standard error | Average | Standard error | p value |
| Blood acetic acid concentration (μM) | 379.5 | 10.69 | 452.9 | 22.60 | 0.01043 |
| Blood propionic acid concentration (μM) | 84.44 | 1.779 | 108.3 | 5.065 | 0.03518 |

As shown in Table 7, the blood acetic acid concentration and the blood propionic acid concentration were significantly increased in the female mice of group B as compared with the female mice of group A.

As shown in Table 8, the blood acetic acid concentration and the blood propionic acid concentration were significantly increased in the unborn babies of female mice of group B as compared with the unborn babies of female mice of group A.

**Claims**

1. An oral administration agent for a pregnant woman comprising an oligosaccharide, for use in increasing a blood short-chain fatty acid concentration in a pregnant woman or an unborn baby.

2. The oral administration agent for a pregnant woman according to claim 1, wherein the oligosaccharide is a fructo-oligosaccharide.

3.  An oral administration agent for a pregnant woman comprising an oligosaccharide, for use in imparting resistance to obesity to an unborn baby.

4.  The oral administration agent for a pregnant woman according to claim 3, wherein the oligosaccharide is a fructo-oligosaccharide.

5.  An oral administration agent for a pregnant woman comprising an oligosaccharide, for use in preventing postnatal obesity, or a disorder resulting from postnatal obesity of an unborn baby.

6.  The oral administration agent for a pregnant woman according to claim 5, wherein the oligosaccharide is a fructo-oligosaccharide.

7.  The oral administration agent for a pregnant woman according to claim 5 or 6, wherein the postnatal obesity is childhood obesity.

8.  The oral administration agent for a pregnant woman according to claim 7, wherein the childhood obesity is early-childhood obesity and/or school-age obesity.

9.  The oral administration agent for a pregnant woman according to any one of claims 5 to 8, wherein the disorder is a disorder of glycometabolism and/or a disorder of lipid metabolism.

10. The oral administration agent for a pregnant woman according to claim 9, wherein the disorder of glycometabolism is abnormal glucose tolerance and/or insulin resistance.

11. The oral administration agent for a pregnant woman according to any one of claims 5 to 8, wherein the disorder is decreased basal metabolism.

12. The oral administration agent for a pregnant woman according to claim 11, wherein the decreased basal metabolism involves decrease in heart rate and/or decrease in body temperature.

## INTERNATIONAL SEARCH REPORT

| International application No. |
|---|
| **PCT/JP2022/021862** |

### A. CLASSIFICATION OF SUBJECT MATTER

*A23L 33/125*(2016.01)i; *A61K 31/702*(2006.01)i; *A61P 3/00*(2006.01)i; *A61P 3/04*(2006.01)i; *A61P 3/06*(2006.01)i; *A61P 3/10*(2006.01)i; *A61P 15/00*(2006.01)i
FI:   A23L33/125; A61P15/00; A61P3/04; A61P3/06; A61P3/10; A61P3/00; A61K31/702

According to International Patent Classification (IPC) or to both national classification and IPC

### B. FIELDS SEARCHED

Minimum documentation searched (classification system followed by classification symbols)

A23L33/125; A61K31/702; A61P3/00; A61P3/04; A61P3/06; A61P3/10; A61P15/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| Y | KIMURA, I. et al. Maternal gut microbiota in pregnancy influences offspring metabolic phenotype in mice. Science. 2020, vol. 367, no. 6481, p.eaaw8429<br>p. 7, section "Dietary fiber intake during pregnancy" | 1-12 |
| Y | 原博, プレバイオティクスから大腸で産生される短鎖脂肪酸の生理効果, 腸内細菌学雑誌, 2002, vol. 16, no. 1, pp. 35-42<br>pp. 35-36, section "How short-chain fatty acids are made", p. 41, section "Short-chain fatty acids and prebiotics", (HARA, Hiroshi. Physiological Effects of Short-Chain Fatty Acid Produced from Prebiotics in the Colon. Journal of Intestinal Microbiology.) | 1-12 |
| Y | JP 2021-17414 A (MEIJI CO LTD) 15 February 2021 (2021-02-15)<br>claims, examples, paragraphs [0026]-[0029] | 1-12 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 July 2022** | **09 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

International application No.

**PCT/JP2022/021862**

| Patent document cited in search report | Publication date (day/month/year) | Patent family member(s) | Publication date (day/month/year) |
|---|---|---|---|
| JP 2021-17414 A | 15 February 2021 | WO 2021/015107 A1 | |

Form PCT/ISA/210 (patent family annex) (January 2015)

## REFERENCES CITED IN THE DESCRIPTION

**Patent documents cited in the description**

- JP 2021017414 A

**Non-patent literature cited in the description**

- **JUNKI MIYAMOTO et al.** *The Lipid,* vol. 27 (2), 2016-4
- Dietary Reference Intakes for Japanese. Japanese Ministry of Health, Labor and Welfare, 2020
- Guidelines for the management of obesity disease in children and adolescents 2017. Life Science Publishing Co., Ltd, 14 April 2017
- Guideline for Early-childhood Obesity. Japan Pediatric Society, nutrition committee, childhood obesity subcommittee, March 2019
- **KIMURA et al.** *Science,* 2020, vol. 367 (6481), eaaw8429
- **MIYAMOTO et al.** *PNAS,* 2019, vol. 116 (47), 23813-23821